# EUROPEAN PATENT SPECIFICATION

(11) **EP 3 549 941 B1**
(45) Date of publication and mention of the grant of the patent: **20.10.2021**
(21) Application number: 19163608.3
(22) Date of filing: 19.03.2019
(51) Int. Cl.: C07D 471/10, C07F 7/08, C07F 7/30, C09K 11/06, H01L 51/50

(54) **ORGANIC ELECTROLUMINESCENCE DEVICE AND POLYCYCLIC COMPOUND FOR ORGANIC ELECTROLUMINESCENCE DEVICE**
ORGANISCHE ELEKTROLUMINESZENZVORRICHTUNG UND POLYCYCLISCHE VERBINDUNG FÜR ORGANISCHE ELEKTROLUMINESZENZVORRICHTUNG
DISPOSITIF ÉLECTROLUMINESCENT ORGANIQUE ET COMPOSÉ POLYCYCLIQUE POUR DISPOSITIF ÉLECTROLUMINESCENT ORGANIQUE

(30) Priority: 05.04.2018 KR 20180039925
(43) Date of publication of application: 09.10.2019
(73) Proprietor: Samsung Display Co., Ltd., Gyeonggi-Do (KR)
(72) Inventor: AKASHI, Nobutaka, Yokohama (JP); SUZAKI, Yuji, Yokohama (JP)
(74) Representative: Shearman, James Ward

(56) References cited:
- WO-A1-2015/158411
- CN-A- 106 831 773
- CN-A- 109 438 446
- KR-A- 20110 120 075
- LIU M. ET AL.: "Horizontally Orientated Sticklike Emitters: Enhancement of Intrinsic Out-Coupling Factor and Electroluminescence Performance", CHEMISTRY OF MATERIALS, vol. 29, no. 20, 5 October 2017 (2017-10-05), pages 8630-8636, XP002790864,

## Description

### BACKGROUND

One or more aspects of embodiments of the present disclosure are related to an organic electroluminescence device (OLED), and a polycyclic compound used for the organic electroluminescence device.

The development of an organic electroluminescence device as an image display device is being actively conducted. The organic electroluminescence device differs from a liquid crystal display device, and is a so-called self-luminescent display device. In a self-luminescent display device, holes and electrons respectively injected from a first electrode and a second electrode recombine in an emission layer, and an organic light-emitting material included in the emission layer may emit light to attain display (e.g., have a display function).

An organic electroluminescence device may include, for example, a first electrode, a hole transport layer on the first electrode, an emission layer on the hole transport layer, an electron transport layer on the emission layer, and a second electrode on the electron transport layer. Holes are injected from the first electrode via the hole transport layer into the emission layer. Electrons are injected from the second electrode via the electron transport layer into the emission layer. The holes and electrons injected into the emission layer may recombine to produce excitons in the emission layer. The organic electroluminescence device may emit light that is generated by excitons transitioning to the ground state. Embodiments of the configuration of the organic electroluminescence device are not limited thereto, and various modifications may be possible.

CN 106 831 773 A relates to a 9,9'-spiral acridine-containing compound and preparation method and application thereof.

WO 2015/158411 A1 relates to hetero-spirobifluorene compounds, methods for their preparation, and organic electroluminescent devices, which contain said hetero-spirobifluorene compounds.

KR 2011 0120075 A relates to spiro compounds and organic light-emitting diodes including the same.

### SUMMARY

One or more aspects of embodiments of the present disclosure provide an organic electroluminescence device (OLED), and a polycyclic compound used in the organic electroluminescence device.

One or more aspects of embodiments of the present disclosure provide an organic electroluminescence device including a first electrode, a hole transport region on the first electrode, an emission layer on the hole transport region, an electron transport region on the emission layer, and a second electrode on the electron transport region, wherein the emission layer includes a polycyclic compound represented by Formula 1:

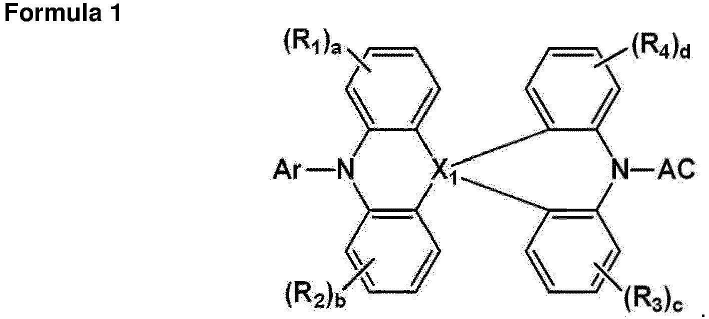

In Formula 1, X₁ is carbon (C), silicon (Si), or germanium (Ge); Ar is a substituted or unsubstituted aryl group having 6 to 30 carbon atoms for forming a ring or a substituted or unsubstituted heteroaryl group having 2 to 30 carbon atoms for forming a ring; R₁ to R₄ are each independently a hydrogen atom (H), a deuterium atom (D), a substituted or unsubstituted alkyl group having 1 to 10 carbon atoms, a substituted or unsubstituted aryl group having 6 to 30 carbon atoms for forming a ring, or a substituted or unsubstituted heteroaryl group having 2 to 30 carbon atoms for forming a ring; "a" to "d" are each independently an integer of 0 to 4, and AC is represented by Formula 3:

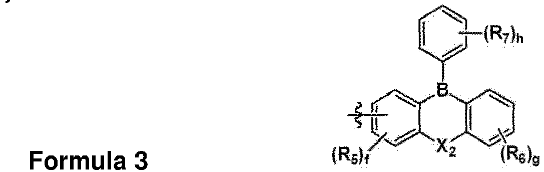

In Formula 3, X₂ is oxygen (O), sulfur (S), or CR₈R₉, where R₅ to R₉ are each independently a hydrogen atom, a deuterium atom, or a substituted or unsubstituted alkyl group having 1 to 5 carbon atoms, or may be combined with an adjacent group to form a hydrocarbon ring or a heterocycle. "f" is an integer of 0 to 3, "g" is an integer of 0 to 4, and "h" is an integer of 0 to 5.

In some embodiments, Formula 3 may be further represented by Formula 3-1 or Formula 3-2:

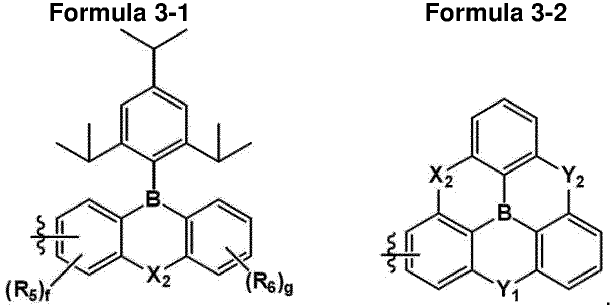

In Formulae 3-1 and 3-2, X₂, R₅, R₆, "f" and "g" are each independently the same as defined in Formula 3; Y₁ and Y₂ are each independently O, S, or CR₂₆R₂₇; and R₂₆ and R₂₇ are each independently a hydrogen atom, a deuterium atom, or a substituted or unsubstituted alkyl group having 1 to 5 carbon atoms.

In some embodiments, in Formula 1, Ar may be a substituted or unsubstituted phenyl group.

In some embodiments, the emission layer may include a host and a dopant, and the dopant may include the polycyclic compound represented by Formula 1.

In some embodiments, the polycyclic compound represented by Formula 1 may be a dopant for thermally activated delayed fluorescence.

In some embodiments, the polycyclic compound represented by Formula 1 may be a blue dopant having a wavelength of less than about 470 nm.

In some embodiments, the hole transport region may include a hole injection layer on the first electrode, a hole transport layer on the hole injection layer, and an electron blocking layer on the hole transport layer.

In some embodiments, the electron transport region may include a hole blocking layer on the emission layer, an electron transport layer on the hole blocking layer, and an electron injection layer on the electron transport layer.

One or more aspects of embodiments of the present disclosure provide the polycyclic compound represented by Formula 1.

At least some of the above and other features of the invention are set out in the claims.

### BRIEF DESCRIPTION OF THE DRAWINGS

The accompanying drawings are included to provide a further understanding of the present disclosure, and are incorporated in and constitute a part of this specification. The drawings illustrate example embodiments of the present disclosure and, together with the description, serve to explain principles of the present disclosure. In the drawings:
FIG. 1 is a cross-sectional view schematically illustrating an organic electroluminescence device according to one or more embodiments of the present disclosure;
FIG. 2 is a cross-sectional view schematically illustrating an organic electroluminescence device according to one or more embodiments of the present disclosure; and
FIG. 3 is a cross-sectional view schematically illustrating an organic electroluminescence device according to one or more embodiments of the present disclosure.

### DETAILED DESCRIPTION

The above objects, other objects, features and advantages of the present disclosure will be better understood from example embodiments with reference to the accompanying drawings. The present disclosure may, however, be embodied in different forms and should not be construed as being limited to the embodiments set forth herein. Rather, example embodiments are provided so that the contents disclosed herein may be thorough and complete, and so that the present disclosure is sufficiently understood by a person skilled in the art.

Like reference numerals refer to like elements in the drawings, and duplicative descriptions may not be provided. In the drawings, the sizes and thicknesses of elements may be enlarged or modified for clarity. It will be understood that although the terms first, second, etc. may be used herein to describe various elements, the elements should not be limited by those terms. These terms are only used to distinguish one element from another element. For example, a first element could be alternatively termed a second element, and similarly, a second element could be alternatively termed a first element. Singular forms used herein are intended to also include the plural forms, unless the context clearly indicates otherwise.

It will be further understood that the terms "comprises" or "comprising," when used in this specification, specify the presence of stated features, numerals, steps, operations, elements, parts, or a combination thereof, but do not preclude the presence or addition of one or more other features, numerals, steps, operations, elements, parts, or a combination thereof. It will be understood that when a layer, a film, a region, a plate, etc. is referred to as being "on" another part, it can be "directly on" the other part, or intervening layers may also be present. When a layer, a film, a region, a plate, etc. is referred to as being "under" another part, it can be "directly under" the other part, or intervening layers may also be present. Further, the use of "may" when describing embodiments of the present disclosure refers to "one or more embodiments of the present disclosure".

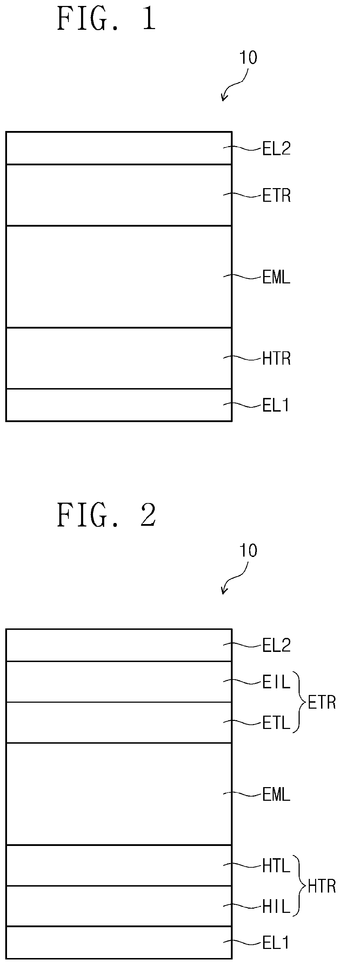

First, organic electroluminescence devices according to example embodiments of the present disclosure will be explained with reference to FIGS. 1-3.

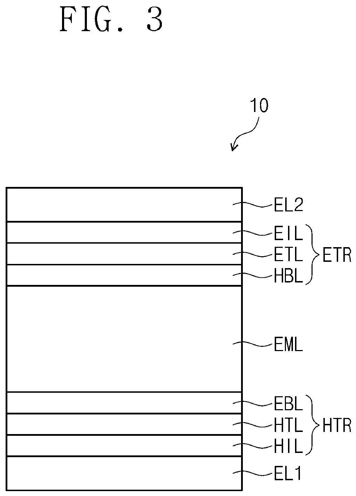

FIG. 1 is a cross-sectional view schematically illustrating an organic electroluminescence device according to one or more embodiments of the present disclosure. FIG. 2 is a cross-sectional view schematically illustrating an organic electroluminescence device according to one or more embodiments of the present disclosure. FIG. 3 is a cross-sectional view schematically illustrating an organic electroluminescence device according to one or more embodiments of the present disclosure.

Referring to FIGS. 1-3, an organic electroluminescence device (OLED) 10 according to one or more embodiments of the present disclosure includes a first electrode EL1, a hole transport region HTR, an emission layer EML, an electron transport region ETR, and a second electrode EL2.

The first electrode EL1 has conductivity (e.g., may be conductive). The first electrode EL1 may be a pixel electrode or an anode. The first electrode EL1 may be a transmissive electrode, a transflective (e.g., semi-transmissive) electrode, or a reflective electrode. When the first electrode EL1 is a transmissive electrode, the first electrode EL1 may include a transparent metal oxide (such as indium tin oxide (ITO), indium zinc oxide (IZO), zinc oxide (ZnO), and/or indium tin zinc oxide (ITZO)). When the first electrode EL1 is a transflective electrode or a reflective electrode, the first electrode EL1 may include silver (Ag), magnesium (Mg), copper (Cu), aluminium (Al), platinum (Pt), palladium (Pd), gold (Au), nickel (Ni), neodymium (Nd), iridium (Ir), chromium (Cr), lithium (Li), calcium (Ca), LiF/Ca, LiF/Al, molybdenum (Mo), titanium (Ti), a compound thereof, or a mixture thereof (for example, a mixture of Ag and Mg). The first electrode EL1 may include a plurality of layers including a reflective layer or transflective layer formed using the above materials, or a transparent layer formed using ITO, IZO, ZnO, or ITZO. For example, the first electrode EL1 may have a three-layer structure of ITO/Ag/ITO, without limitation.

The thickness of the first electrode EL1 may be about 1,000 Å to about 10,000 Å, for example, about 1,000 Å to about 3,000 Å.

The hole transport region HTR is on the first electrode EL1. The hole transport region HTR may include at least one of a hole injection layer HIL, a hole transport layer HTL, a hole buffer layer, and an electron blocking layer EBL. The thickness of the hole transport region HTR may be, for example, about 100 Å to about 1,500 Å. In some embodiments, for example, the hole transport region HTR may include a hole injection layer HIL and a hole transport layer HTL, as shown in FIG. 2. In some embodiments, the hole transport region HTR may include a hole injection layer HIL, a hole transport layer HTL, and an electron blocking layer EBL, as shown in FIG. 3.

The hole transport region HTR may be a single layer formed using a single material, a single layer formed using a plurality of different materials, or a multilayer structure including a plurality of layers formed using a plurality of different materials.

For example, the hole transport region HTR may have the structure of a single layer (such as a hole injection layer HIL and a hole transport layer HTL, e.g., together), and may have a structure of a single layer formed using a hole injection material and a hole transport material. In some embodiments, the hole transport region HTR may have a structure of a single layer formed using a plurality of different materials, or a structure laminated on the first electrode EL1, for example, including a hole injection layer HIL/hole transport layer HTL, a hole injection layer HIL/hole transport layer HTL/hole buffer layer, a hole injection layer HIL/hole buffer layer, a hole transport layer HTL/hole buffer layer, or a hole injection layer HIL/hole transport layer HTL/electron blocking layer EBL, without limitation.

The hole transport region HTR may be formed using one or more suitable methods (such as a vacuum deposition method, a spin coating method, a cast method, a Langmuir-Blodgett (LB) method, an inkjet printing method, a laser printing method, and/or a laser induced thermal imaging (LITI) method).

The hole injection layer HIL may include, for example, a phthalocyanine compound (such as copper phthalocyanine); N,N'-diphenyl-N,N'-bis-[4-(phenyl-m-tolyl-amino)-phenyl]-biphenyl-4,4'-diamine (DNTPD), 4,4',4"-tris[(3-methylphenyl)phenylamino] triphenylamine (m-MTDATA), 4,4',4"-tris(N,N-diphenylamino)triphenylamine (TDATA), 4,4',4"-tris{N-(2-naphthyl)-N-phenylamino}-triphenylamine (2-TNATA), poly(3,4-ethylenedioxythiophene)/poly(4-styrenesulfonate) (PEDOT/PSS), polyaniline/dodecylbenzenesulfonic acid (PANI/DBSA), polyaniline/camphor sulfonic acid (PANI/CSA), polyaniline/poly(4-styrenesulfonate) (PANI/PSS), N,N'-dinaphthalene-1-yl)-N,N'-diphenyl-benzidine (NPB), triphenylamine-containing polyether ketone (TPAPEK), 4-isopropyl-4'-methyldiphenyliodonium tetrakis(pentafluorophenyl)borate, dipyrazino[2,3-f: 2',3'-h] quinoxaline-2,3,6,7,10,11-hexacarbonitrile (HAT-CN), etc.

The hole transport layer HTL may include, for example, carbazole derivatives (such as N-phenyl carbazole and/or polyvinyl carbazole), fluorene-based derivatives, N,N'-bis(3-methylphenyl)-N,N'-diphenyl-[1,1 -biphenyl]-4,4'-diamine (TPD), triphenylamine-based derivatives (such as 4,4',4"-tris(N-carbazolyl)triphenylamine (TCTA)), N,N'-di(1-naphthalene-1-yl)-N,N'-diphenyl-benzidine (NPB), 4,4'-cyclohexylidene bis[N,N-bis(4-methylphenyl)benzenamine] (TAPC), 4,4'-bis[N,N'-(3-tolyl)amino]-3,3'-dimethylbiphenyl (HMTPD), 1,3-bis(N-carbazolyl)benzene (mCP), etc.

The thickness of the hole transport region HTR may be about 100 Å to about 10,000 Å, for example, about 100 Å to about 7000 Å, about 100 Å to about 5000 Å, about 100 Å to about 3000 Å, or about 100 Å to about 1,000 Å. When the hole transport region HTR includes both (e.g., simultaneously) a hole injection layer HIL and a hole transport layer HTL, the thickness of the hole injection layer HIL may be about 100 Å to about 10,000 Å, for example, about 100 Å to about 7,000 Å, about 100 Å to about 5,000 Å, about 100 Å to about 3,000 Å, about 100 Å to about 2,000 Å, about 100 Å to about 1,000 Å or about 100 Å to about 200 Å, and the thickness of the hole transport layer HTL may be about 30 Å to about 1,000 Å, about 100 Å to about 1,000 Å, about 500 Å to about 1000 Å, about 600 Å to about 1000 Å, or about 700 Å to about 900 Å. When the thicknesses of the hole transport region HTR, the hole injection layer HIL, and the hole transport layer HTL each satisfy the above-described ranges, satisfactory hole transport properties may be obtained without a substantial increase in driving voltage.

The hole transport region HTR may further include a charge generating material in addition to the above-described materials to increase conductivity. The charge generating material may be dispersed uniformly or non-uniformly in the hole transport region HTR. The charge generating material may be, for example, a p-dopant. The p-dopant may be a quinone derivative, a metal oxide, or a cyano group-containing compound, without limitation. Non-limiting examples of the p-dopant may include quinone derivatives (such as tetracyanoquinodimethane (TCNQ) and/or 2,3,5,6-tetrafluoro-7,7,8,8-tetracyanoquinodimethane (F4-TCNQ)), metal oxides (such as tungsten oxide and/or molybdenum oxide).

As described above, the hole transport region HTR may further include at least one of a hole buffer layer and an electron blocking layer EBL, in addition to the hole injection layer HIL and the hole transport layer HTL. The hole buffer layer may compensate for a resonance distance according to the wavelength of light emitted from the emission layer EML (e.g., be used to adjust the optical resonance distance to match the wavelength of light emitted from the emission layer) and to increase the light emission efficiency of the device. Materials included in the hole transport region HTR may also be included in the hole buffer layer. The electron blocking layer EBL may prevent or reduce electron injection from the electron transport region ETR to the hole transport region HTR.

The emission layer EML may be on the hole transport region HTR. The emission layer EML may have a thickness of about 100 Å to about 1,000 Å, about 100 Å to about 800 Å, about 100 Å to about 700 Å, about 100 Å to about 600 Å, about 100 Å to about 500 Å, about 100 Å to about 400 Å or about 100 Å to about 300 Å. The emission layer EML may be a single layer formed using a single material, a single layer formed using a plurality of different materials, or a multilayer structure having a plurality of layers formed using a plurality of different materials.

The emission layer EML includes a polycyclic compound according to one or more embodiments of the present disclosure. Hereinafter, the polycyclic compound according to one or more embodiments of the present disclosure will be explained in more detail.

In the description, 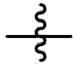 refers to a part to be connected (e.g., a connection point to another moiety or formula structure).

In the description, "substituted or unsubstituted" may refer to substitution with at least one substituent selected from the group consisting of a deuterium atom, a halogen atom, a cyano group, a nitro group, a nitro group, an amino group, a silyl group, a boron group, a phosphine oxide group, a phosphine sulfide group, an alkyl group, an alkenyl group, an aryl group, and a heterocycle, or absence of the above substituents (e.g., substitution with hydrogen atoms only). In some embodiments, each of the above substituents may be further substituted or unsubstituted. For example, a biphenyl group may be interpreted as an aryl group in itself, or as a phenyl group substituted with a phenyl group. The term "heterocycle" as used herein may refer to aliphatic heterocycles and aromatic heterocycles (heteroaryl group).

In some embodiments, "substituted or unsubstituted" may refer to being substituted with one or more substituents selected from the group consisting of a deuterium atom, a halogen atom, a cyano group, an alkyl group, an aryl group, and a heterocycle, as well as being unsubstituted.

In the description, the term "halogen atom" may refer to a fluorine atom (F), a chlorine atom (CI), a bromine atom (Br), and/or an iodine atom (I).

In the description, the term "alkyl" (e.g., "alkyl group") may refer to a linear, branched, or cyclic alkyl group. The carbon number of the alkyl may be 1 to 30, 1 to 20, 1 to 10, or 1 to 5. Non-limiting examples of an alkyl group may include methyl, ethyl, n-propyl, isopropyl, n-butyl, s-butyl, t-butyl, i-butyl, 2-ethylbutyl, 3,3-dimethylbutyl, n-pentyl, i-pentyl, neopentyl, t-pentyl, cyclopentyl, 1-methylpentyl, 3-methylpentyl, 2-ethylpentyl, 4-methyl-2-pentyl, n-hexyl, 1-methylhexyl, 2-ethylhexyl, 2-butylhexyl, cyclohexyl, 4-methylcyclohexyl, 4-t-butylcyclohexyl, n-heptyl, 1-methylheptyl, 2,2-dimethylheptyl, 2-ethylheptyl, 2-butylheptyl, n-octyl, t-octyl, 2-ethyloctyl, 2-butyloctyl, 2-hexyloctyl, 3,7-dimethyloctyl, cyclooctyl, n-nonyl, n-decyl, adamantyl, 2-ethyldecyl, 2-butyldecyl, 2-hexyldecyl, 2-octyldecyl, n-undecyl, n-dodecyl, 2-ethyldodecyl, 2-butyldodecyl, 2-hexyldocecyl, 2-octyldodecyl, n-tridecyl, n-tetradecyl, n-pentadecyl, n-hexadecyl, 2-ethylhexadecyl, 2-butylhexadecyl, 2-hexylhexadecyl, 2-octylhexadecyl, n-heptadecyl, n-octadecyl, n-nonadecyl, n-eicosyl, 2-ethyleicosyl, 2-butyleicosyl, 2-hexyleicosyl, 2-octyleicosyl, n-henicosyl, n-docosyl, n-tricosyl, n-tetracosyl, n-pentacosyl, n-hexacosyl, n-heptacosyl, n-octacosyl, n-nonacosyl, n-triacontyl, etc.

In the description, the term "alkenyl group" may refer to a linear chain (e.g., linear alkene) or a branched chain (e.g., branched alkene). The carbon number is not specifically limited, and for example, may be 2 to 30, 2 to 20, or 2 to 10. Non-limiting examples of the alkenyl group may include vinyl, 1-butenyl, 1-pentenyl, 1,3-butadienyl aryl, styrenyl, styrylvinyl, etc. However, embodiments of the present disclosure are not limited thereto.

In the description, the term "aryl group" refers to a functional group or substituent derived from an aromatic hydrocarbon ring. The aryl group may be a monocyclic aryl group or a polycyclic aryl group. The number of carbon atoms for forming a ring in the aryl group may be 6 to 60, 6 to 30, 6 to 20, or 6 to 15. Non-limiting examples of the aryl group may include phenyl, naphthyl, fluorenyl, anthracenyl, phenanthryl, biphenyl, terphenyl, quaterphenyl, quinqphenyl, sexiphenyl, biphenylene, triphenylene, pyrenyl, benzofluoranthenyl, chrysenyl, etc.

In the description, the fluorenyl group may be substituted (e.g., at the 9H position), and two substituents of the fluorenyl group may be combined with each other to form a spiro structure. Non-limiting examples of substituted fluorenyl groups are shown below. However, embodiments of the present disclosure are not limited thereto.

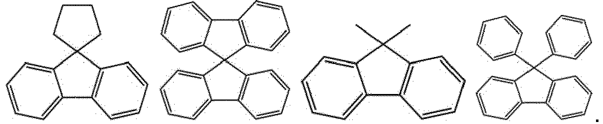

In the description, the term "heterocycle" may refer to a cyclic structure including at least one of boron (B), O, N, phosphorus (P), silicon (Si), or S as a heteroatom. When the heterocycle includes two or more heteroatoms, the two or more heteroatoms may be the same or different. The heterocycle may be heteroaromatic, and may be a monocyclic heteroaryl group or a polycyclic heteroaryl group. The number of carbons for forming a ring in the heterocycle may be 2 to 30, 2 to 20, or 2 to 10. Non-limiting examples of the polycycle (e.g., heterocycle) may include thiophene, furan, pyrrole, imidazole, thiazole, oxazole, oxadiazole, triazole, pyridine, bipyridine, pyrimidine, triazine, triazole, acridyl, pyridazine, pyrazinyl, quinoline, quinazoline, quinoxaline, phenoxazine, phthalazine, pyrido pyrimidine, pyrido pyrazine, pyrazino pyrazine, isoquinoline, indole, carbazole, N-arylcarbazole, N-heteroarylcarbazole, N-alkylcarbazole, benzoxazole, benzimidazole, benzothiazole, benzocarbazole, benzothiophene, dibenzothiophene, thienothiophene, benzofuran, phenanthroline, thiazole, isooxazole, oxadiazole, thiadiazole, phenothiazine, dibenzosilole, dibenzofuran, etc.

In the description, the term "silyl group" may refer to an alkyl silyl group and/or an aryl silyl group. Non-limiting examples of the silyl group may include trimethylsilyl, triethylsilyl, t-butyldimethylsilyl, vinyldimethylsilyl, propyldimethylsilyl, triphenylsilyl, diphenylsilyl, phenylsilyl, etc. However, embodiments of the present disclosure are not limited thereto.

In the description, the boron group includes an alkyl boron group and an aryl boron group. Non-limiting examples of the boron group may include trimethylboron, triethylboron, t-butyldimethylboron, triphenylboron, diphenylboron, phenylboron, etc. However, embodiments of the present disclosure are not limited thereto.

In the description, the carbon number of the amino group is not specifically limited, and for example, may be 1 to 30. The amino group may include an alkyl amino group and an aryl amino group. Non-limiting examples of the amino group include a methylamino group, a dimethylamino group, a phenylamino group, a diphenylamino group, a naphthylamino group, a 9-methyl-anthracenylamino group, a triphenylamino group, etc.

In the description, the phosphine oxide group may be substituted with at least one of an alkyl group or an aryl group. Non-limiting examples of the phosphine oxide group include a phenyl phosphine oxide group, diphenyl phosphine oxide group, etc.

In the description, the phosphine sulfide group may be substituted with at least one of an alkyl group or an aryl group.

The polycyclic compound according to one or more embodiments of the present disclosure is represented by Formula 1:
**Formula 1**

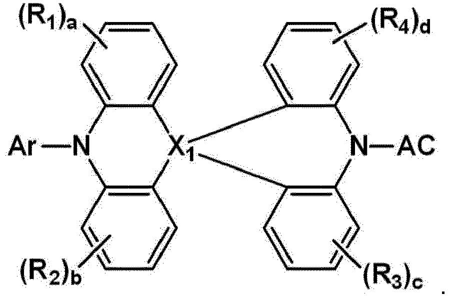

In Formula 1, X₁ is C, Si, or Ge; Ar is a substituted or unsubstituted aryl group having 6 to 30 carbon atoms for forming a ring, or a substituted or unsubstituted heteroaryl group having 2 to 30 carbon atoms for forming a ring; R₁ to R₄ are each independently a hydrogen atom (H), a deuterium atom (D), a substituted or unsubstituted alkyl group having 1 to 10 carbon atoms, a substituted or unsubstituted aryl group having 6 to 30 carbon atoms for forming a ring, or a substituted or unsubstituted heteroaryl group having 2 to 30 carbon atoms for forming a ring; "a" to "d" are each independently an integer of 0 to 4, and AC is represented by Formula 3:

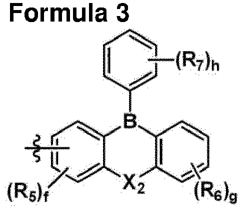

In Formula 3, X₂ is O, S or CR₈R₉; R₅ to R₉ are each independently a hydrogen atom, a deuterium atom, or a substituted or unsubstituted alkyl group having 1 to 5 carbon atoms, or may be combined with an adjacent group to form a hydrocarbon ring or a heterocycle; "f" is an integer of 0 to 3, "g" is an integer of 0 to 4, and "h" is an integer of 0 to 5.

In Formula 1, if "a" is 2 or more, a plurality of R₁ groups are the same or different; if "b" is 2 or more, a plurality of R₂ groups are the same or different; if "c" is 2 or more, a plurality of R₃ groups are the same or different; and if "d" is 2 or more, a plurality of R₄ groups are the same or different.

In Formula 1, if "a" is 0, R₁ may not be a hydrogen atom; if "b" is 0, R₂ may not be a hydrogen atom; if "c" is 0, R₃ may not be a hydrogen atom; and if "d" is 0, R₄ may not be a hydrogen atom.

For example, each of "a" to "d" may be 0. However, embodiments of the present disclosure are not limited thereto. In order to control the energy level of a polycyclic compound, etc., at least one of "a" to "d" may be 1 or more, and a substituent other than a hydrogen atom may be introduced.

In Formula 1, Ar may be a substituted or unsubstituted aryl group having 6 to 20 carbon atoms. For example, Ar may be a substituted or unsubstituted phenyl group, a substituted or unsubstituted naphthyl group, a substituted or unsubstituted biphenyl group, a substituted or unsubstituted anthracenyl group, a substituted or unsubstituted phenanthryl group, or a substituted or unsubstituted terphenyl group. For example, Ar may be a substituted or unsubstituted phenyl group. For example, Ar may be an unsubstituted phenyl group.

In Formula 1, R₁ to R₄ may each independently be a hydrogen atom, a deuterium atom, a substituted or unsubstituted alkyl group having 1 to 5 carbon atoms, a substituted or unsubstituted aryl group having 6 to 20 carbon atoms for forming a ring, or a substituted or unsubstituted heteroaryl group having 2 to 20 carbon atoms for forming a ring.

AC in Formula 1 is an electron acceptor, and the structure excluding AC in Formula 1 is an electron donor. For example, the polycyclic compound according to one or more embodiments of the present disclosure includes an electron acceptor and an electron donor in one molecule.

In Formula 3, if "f" is 2 or more, a plurality of R₅ groups are the same or different; if "g" is 2 or more, a plurality of R₆ groups are the same or different; and if "h" is 2 or more, a plurality of R₇ groups are the same or different.

In Formula 3, if "f" is 0, R₅ may not be a hydrogen atom; if "g" is 0, R₆ may not be a hydrogen atom; and if "h" is 0, R₇ may not be a hydrogen atom.

For example, "h" may be 1 or more, and R₇ may be a substituted or unsubstituted alkyl group having 1 to 5 carbon atoms. In some embodiments, Formula 3 may be further represented by Formula 3-1:

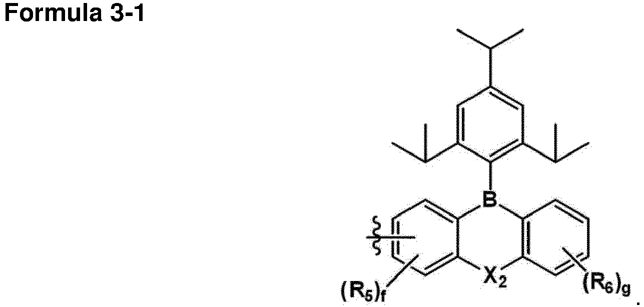

In Formula 3-1, X₂, R₅, R₆, "f" and "g" are each independently the same as defined in Formula 3. In some embodiments, Formula 3-1 may be further represented by Formula 3-1-1 or 3-1-2:

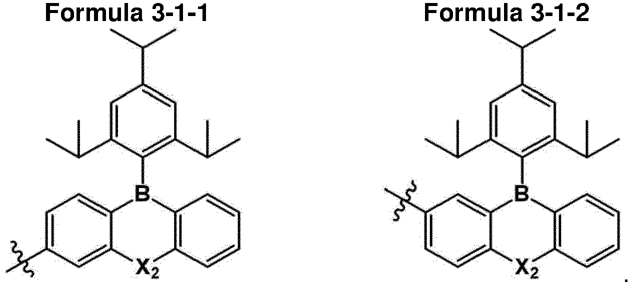

In Formulae 3-1-1 and 3-1-2, X₂ is the same as defined in Formula 3.

In Formula 3, X₂ may be O or S. In Formula 3, X₂ may be CR₈R₉, where R₅ and R₉ may each independently be a substituted or unsubstituted methyl group.

As described above, in Formula 3, R₅ to R₇ may be combined with an adjacent group to form a hydrocarbon ring or a heterocycle. In some embodiments, for example, Formula 3 may be further represented by Formula 3-2:

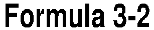

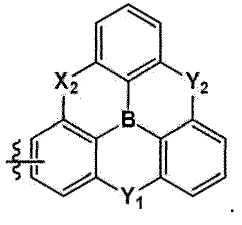

In Formula 3-2, X₂ is the same as defined in Formula 3; Y₁ and Y₂ are each independently O, S or CR₂₆R₂₇; and R₂₆ and R₂₇ are each independently a hydrogen atom, a deuterium atom, or a substituted or unsubstituted alkyl group having 1 to 5 carbon atoms. For example, each of R₂₆ and R₂₇ may be a substituted or unsubstituted methyl group. For example, each of R₂₆ and R₂₇ may be an unsubstituted methyl group.

In Formula 3-2, at least one of Y₁ and Y₂ may be CR₂₆R₂₇.

In some embodiments, Formula 3-2 may be further represented by one of Formulae 3-2-1 to 3-2-5:

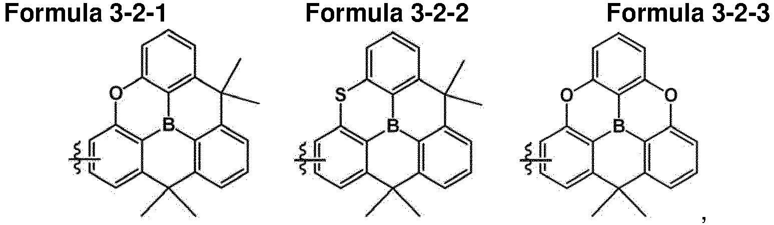

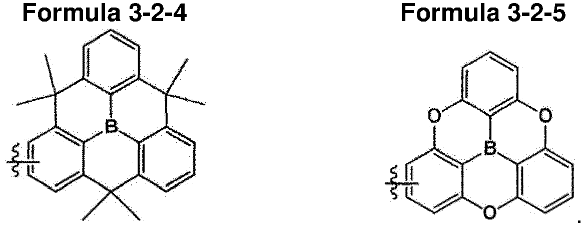

The polycyclic compound represented by Formula 1 may be at least one selected from the compounds represented in Compound Group 1, but embodiments of the present disclosure are not limited thereto:

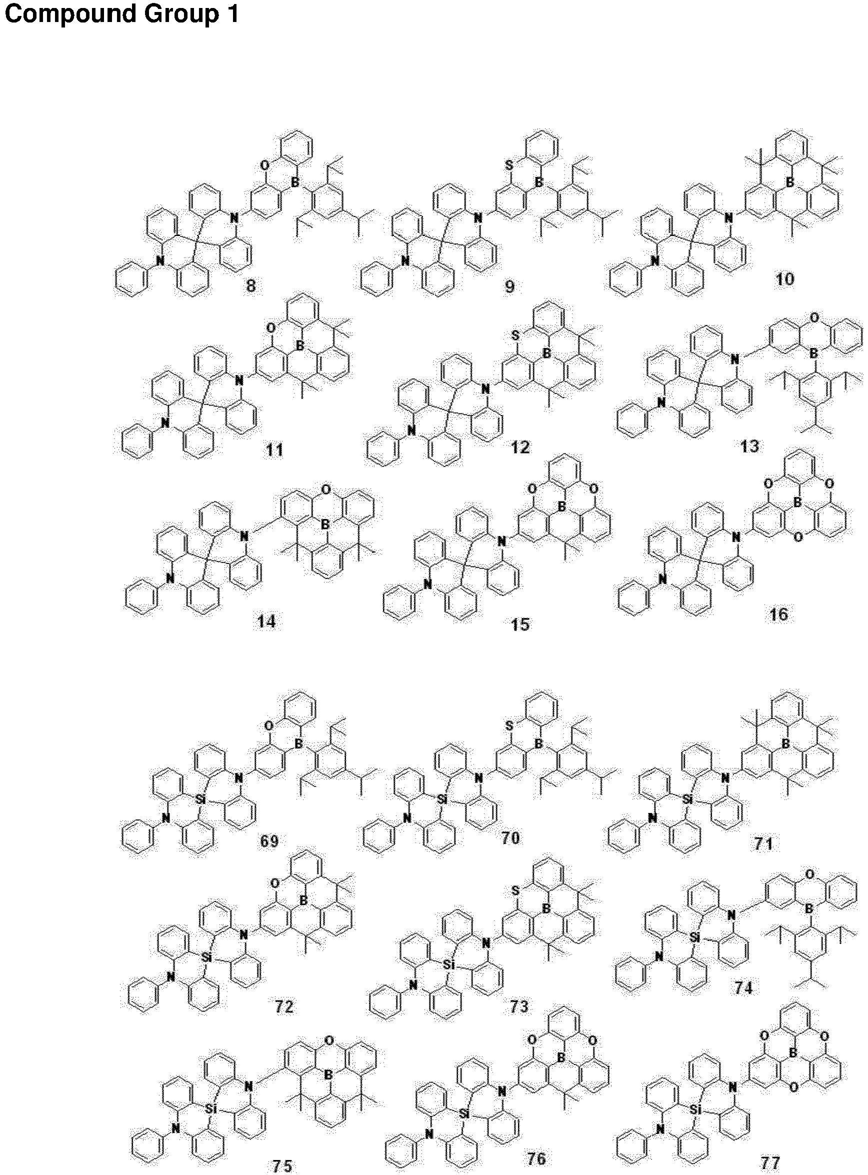

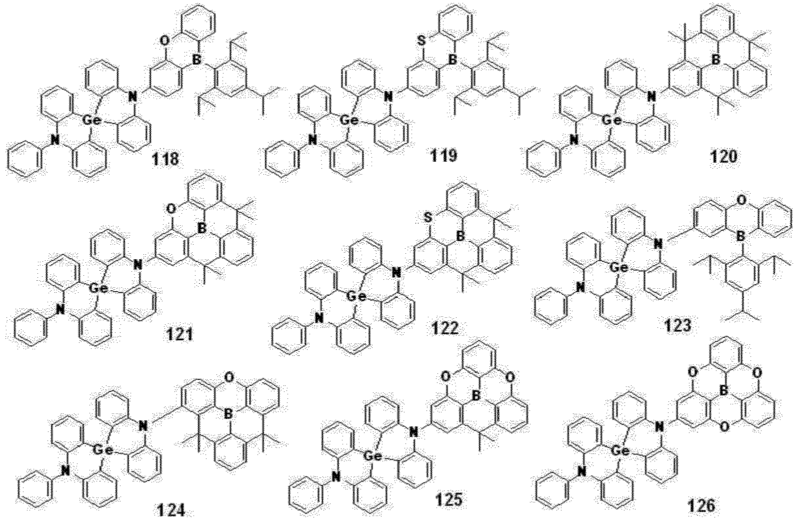

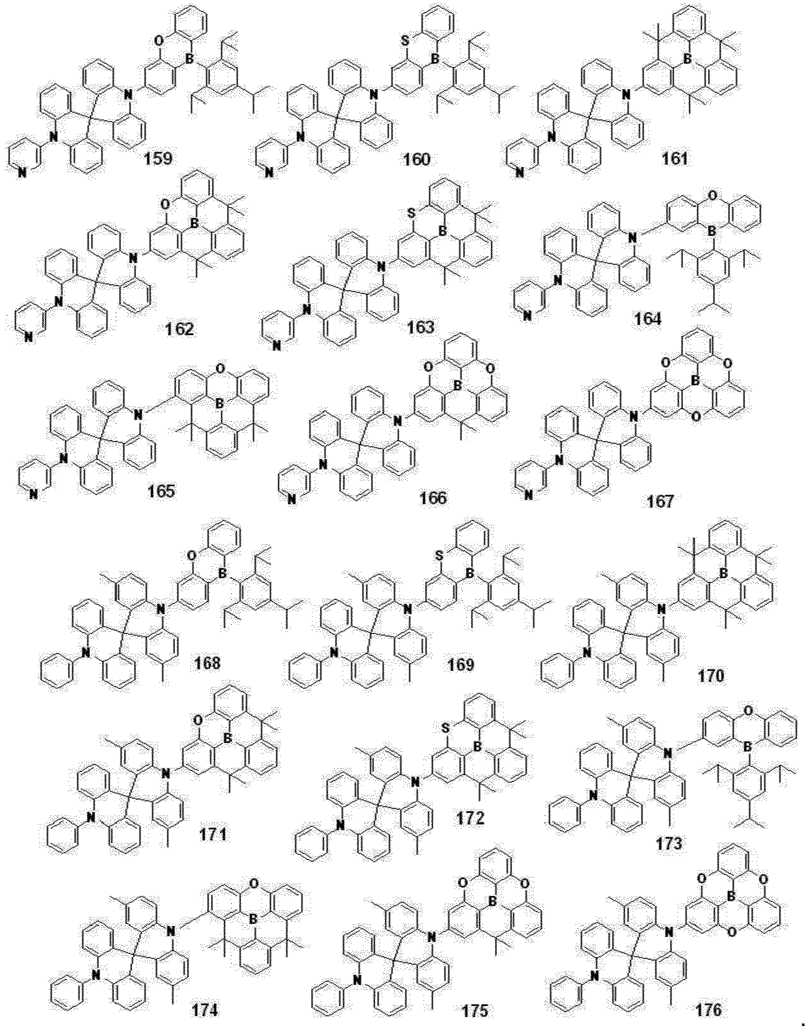

The polycyclic compound according to one or more embodiments of the present disclosure may have an energy level difference between a singlet energy level and a triplet energy level of about 0.2 eV or less, and as a result, may be utilized as a material for thermally activated delayed fluorescence. The polycyclic compound according to one or more embodiments of the present disclosure may be used as a material for an organic electroluminescence device to contribute to an increase in efficiency.

An organic electroluminescence device will be explained by reference to FIGS. 1-3.

The emission layer EML may include one or two or more kinds of structures of the polycyclic compound represented by Formula 1. The emission layer EML may further include other suitable materials in addition to the polycyclic compound represented by Formula 1.

The emission layer EML may include a host and a dopant, and the dopant may include the polycyclic compound represented by Formula 1. The polycyclic compound according to one or more embodiments of the present disclosure may be included in the emission layer EML as a dopant for thermally activated delayed fluorescence. The polycyclic compound according to one or more embodiments of the present disclosure may be included in the emission layer EML as a dopant for emitting blue light having a wavelength of about 470 nm or less, and for example, may be included in the emission layer EML as a dopant for emitting deep blue light having a wavelength of about 440 nm to about 470 nm, or about 450 nm to about 470 nm.

The host material may include suitable materials available in the art, without limitation. For example, at least one of bis[2-(diphenylphosphino)phenyl] ether oxide (DPEPO), 4,4'-bis(carbazol-9-yl)biphenyl (CBP), 1,3-bis(carbazol-9-yl)benzene (mCP), 2,8-bis(diphenylphosphoryl)dibenzo[b,d]furan (PPF), 4,4',4"-tris(carbazol-9-yl)-triphenylamine (TCTA), and 1,3,5-tris(N-phenylbenzimidazole-2-yl)benzene (TPBi) may be included. However, embodiments of the present disclosure are not limited thereto. For example, tris(8-hydroxyquinolino)aluminum (Alq₃), 4,4'-bis(N-carbazolyl)-1,1'-biphenyl (CBP), poly(N-vinylcarbazole) (PVK), 9,10-di(naphthalene-2-yl)anthracene (ADN), 4,4',4"-tris(carbazol-9-yl)-triphenylamine (TCTA), 1,3,5-tris(N-phenylbenzimidazole-2-yl)benzene (TPBi), 3-tert-butyl-9,10-di(naphth-2-yl)anthracene (TBADN), distyrylarylene (DSA), 4,4'-bis(9-carbazolyl)-2,2'-dimethyl-biphenyl (CDBP), 2-methyl-9,10-bis(naphthalen-2-yl)anthracene (MADN), bis[2-(diphenylphosphino)phenyl]ether oxide (DPEPO), hexaphenyl cyclotriphosphazene (CP1), 1,4-bis(triphenylsilyl)benzene (UGH2), hexaphenylcyclotrisiloxane (DPSiO₃), octaphenylcyclotetra siloxane (DPSiO₄), 2,8-bis(diphenylphosphoryl)dibenzofuran (PPF), etc., may be used or included as the host material.

For example, the emission layer EML may further include at least one of N,N,N',N'-tetraphenyl-pyrene-1,6-diamine (TPD), 4,4'-bis(2-(9-ethyl-9H-carbazol-3-yl)vinyl)-1,1'-biphenyl (BCzVBi); 4,4'-bis(9-ethyl-3-carbazovinylene)-1,1'-biphenyl, 10-phenyl-10H,10'H-spiro[acridine-9,9'-anthracene]-10'-one (ACRSA), 3,4,5,6-tetra-9H-carbazol-9-yl-1,2-benzenedicarbonitrile (4CzPN), 2,4,5,6-tetra-9H-carbazol-9-yl-isophthalonitrile (4CzIPN), bis[4-9,9-dimethyl-9,10-dihydroacridine]phenyl]sulfone (DMAC-DPS), and 2-phenoxazine-4,6-diphenyl-1,3,5-triazine (PSZ-TRZ) as a dopant. In some embodiments, the emission layer EML may further include styryl derivatives (for example, 1,4-bis[2-(3-N-ethylcarbazoryl)vinyl]benzene (BCzVB), 4-(di-p-tolylamino)-4'-[(di-p-tolylamino)styryl]stilbene (DPAVB), and N-(4-((E)-2-(6-((E)-4-(diphenylamino)styryl)naphthalen-2-yl)vinyl)phenyl)-N-phenylbenzenamine (N-BDAVBi)), perylene and derivatives thereof (for example, 2,5,8,11-tetra-t-butylperylene (TBP)), pyrene and the derivatives thereof (for example, 1,1 -dipyrene, 1,4-dipyrenylbenzene, 1,4-bis(N,N-diphenylamino)pyrene), etc., as dopant materials.

The emission layer EML may be a blue emission layer to emit blue light. The emission layer EML may be an emission layer to emit light in a wavelength of about 480 nm or less, or about 470 nm or less. The emission layer EML may be a fluorescence emission layer radiating fluorescence. The emission layer EML may be a delayed fluorescence emission layer radiating delayed fluorescence.

The electron transport region ETR is on the emission layer EML. The electron transport region ETR may include at least one of a hole blocking layer HBL, an electron transport layer ETL, or an electron injection layer EIL, without limitation. In some embodiments, for example, the electron transport region ETR may include an electron injection layer EIL and an electron transport layer ETL, as shown in FIG. 2. In some embodiments, the electron transport region ETR may include an electron injection layer EIL, an electron transport layer ETL, and hole blocking layer EBL, as shown in FIG. 3.

The electron transport region ETR may have or be a single layer formed using a single material, a single layer formed using a plurality of different materials, or a multilayer structure having a plurality of layers formed using a plurality of different materials.

For example, the electron transport region ETR may have a single layer structure of an electron injection layer EIL or an electron transport layer ETL, or a single layer structure formed using an electron injection material and an electron transport material. Further, the electron transport region ETR may have a single layer structure having a plurality of different materials, or a structure laminated from the emission layer EML of electron transport layer ETL/electron injection layer EIL, or hole blocking layer HBL/electron transport layer ETL/electron injection layer EIL, without limitation. The thickness of the electron transport region ETR may be, for example, from about 1,000 Å to about 1,500 Å.

The electron transport region ETR may be formed using one or more suitable methods (such as a vacuum deposition method, a spin coating method, a cast method, a Langmuir-Blodgett (LB) method, an inkjet printing method, a laser printing method, and/or a laser induced thermal imaging (LITI) method).

If the electron transport region ETR includes an electron transport layer ETL, the electron transport region ETR may include an anthracene-based compound. However, embodiments of the present disclosure are not limited thereto. The electron transport region ETR may include, for example, tris(8-hydroxyquinolinato)aluminum (Alq₃), 1,3,5-tri[(3-pyridyl)-phen-3-yl]benzene, 2,4,6-tris(3'-(pyridin-3-yl)biphenyl-3-yl)-1 ,3,5-triazine, 2-(4-(N-phenylbenzoimidazolyl-1-ylphenyl)-9,10-dinaphthylanthracene, 1,3,5-tri(1-phenyl-1H-benzo[d]imidazol-2-yl)phenyl (TPBi), 2,9-dimethyl-4,7-diphenyl-1,10-phenanthroline (BCP), 4,7-diphenyl-1,10-phenanthroline (Bphen), 3-(4-biphenylyl)-4-phenyl-5-tert-butylphenyl-1,2,4-triazole (TAZ), 4-(naphthalen-1-yl)-3,5-diphenyl-4H-1,2,4-triazole (NTAZ), 2-(4-biphenylyl)-5-(4-tert-butylphenyl)-1,3,4-oxadiazole (tBu-PBD), bis(2-methyl-8-quinolinolato-N1,08)-(1,1'-biphenyl-4-olato)aluminum (BAlq), beryllium bis(benzoquinolin-10-olate) (Bebq₂), 9,10-di(naphthalene-2-yl)anthracene (ADN), and/or a mixture thereof. The thickness of the electron transport layer ETL may be about 100 Å to about 1,000 Å, for example, about 100 Å to about 700 Å, about 100 Å to about 600 Å, about 150 Å to about 600 Å, about 150 Å to about 500 Å or about 250 Å to about 350 Å. When the thickness of the electron transport layer ETL satisfies the above-described range, satisfactory electron transport properties may be obtained without a substantial increase in driving voltage.

When the electron transport region ETR includes an electron injection layer EIL, the electron transport layer ETL may use LiF, lithium quinolate (LiQ), Li₂O, BaO, NaCl, CsF, a lanthanide metal (such as ytterbium (Yb)), or a metal halide (such as RbCI and/or Rbl). However, embodiments of the present disclosure are not limited thereto. The electron injection layer EIL may also be formed using a mixture of an electron transport material and an insulating organometallic salt. The organometallic salt may be a material having an energy band gap of about 4 eV or more. Non-limiting examples of the organometallic salt may include metal acetates, metal benzoates, metal acetoacetates, metal acetylacetonates, and metal stearates. The thickness of the electron injection layer EIL may be about 1 Å to about 100 Å, about 3 Å to about 90 Å or about 3 Å to about 10 Å. When the thickness of the electron injection layer EIL satisfies the above-described range, satisfactory electron injection properties may be obtained without substantial increase of a driving voltage.

The electron transport region ETR may include a hole blocking layer HBL as described above. The hole blocking layer may include, for example, 2,9-dimethyl-4,7-diphenyl-1,10-phenanthroline (BCP), and/or 4,7-diphenyl-1,10-phenanthroline (Bphen). However, embodiments of the present disclosure are not limited thereto.

The second electrode EL2 may be on the electron transport region ETR. The second electrode EL2 may be a common electrode or a cathode. The second electrode EL2 may be a transmissive electrode, a transflective (e.g., semi-transmissive) electrode, or a reflective electrode. When the second electrode EL2 is a transmissive electrode, the second electrode EL2 may include a transparent metal oxide, for example, ITO, IZO, ZnO, ITZO, etc.

When the second electrode EL2 is a transflective electrode or a reflective electrode, the second electrode EL2 may include Ag, Mg, Cu, Al, Pt, Pd, Au, Ni, Nd, Ir, Cr, Li, Ca, LiF/Ca, LiF/AI, Mo, Ti, a compound including one or more of the listed metals, or a mixture thereof (for example, a mixture of Ag and Mg). The second electrode EL2 may have a multilayered structure including a reflective layer or a transflective layer formed using the above-described materials and a transparent conductive layer formed using ITO, IZO, ZnO, ITZO, etc.

In some embodiments, the second electrode EL2 may be connected with (to) an auxiliary electrode. When the second electrode EL2 is connected with the auxiliary electrode, the resistance of the second electrode EL2 may decrease.

In the organic electroluminescence device 10, during application of a voltage to each of the first electrode EL1 and second electrode EL2, holes injected from the first electrode EL1 may move via the hole transport region HTR to the emission layer EML, and electrons injected from the second electrode EL2 may move via the electron transport region ETR to the emission layer EML. The electrons and the holes may recombine in the emission layer EML to produce excitons, and the excitons may emit light when they transition from an excited state to the ground state.

When the organic electroluminescence device 10 is a top emission type (top emission OLED), the first electrode EL1 may be a reflective electrode and the second electrode EL2 may be a transmissive electrode or a transflective electrode. When the organic electroluminescence device 10 is a bottom emission type (bottom emission OLED), the first electrode EL1 may be a transmissive electrode or a transflective electrode and the second electrode EL2 may be a reflective electrode.

The organic electroluminescence device 10 according to one or more embodiments of the present disclosure may include the polycyclic compound as a material for an emission layer EML, and thus, high efficiency may be achieved.

Hereinafter, the present disclosure will be explained in more detail with reference to example preparation methods, examples, and comparative examples. The following examples are illustrations to assist in the understanding of the present disclosure, and the scope of embodiments of the present disclosure is not limited thereto.

### Synthetic Examples

The polycyclic compound according to one or more embodiments of the present disclosure may be synthesized, for example, as follows. However, embodiments of the present disclosure are not limited thereto.

### 1. Synthesis of Compound 8

Compound 8 according to one or more embodiments of the present disclosure may be synthesized, for example, as follows.

### Synthesis of Intermediate 1A

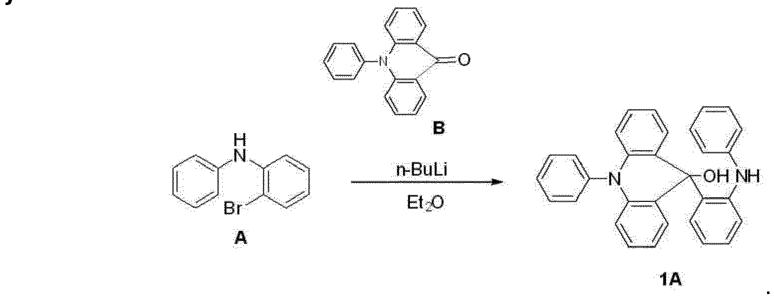

2.0 g of Compound A and 30 mL of dehydrated diethyl ether were added to a 200 mL, three-neck flask under an argon (Ar) atmosphere, and 10 mL of 1.6 M n-BuLi was added dropwise thereto at room temperature, followed by stirring at room temperature for about 2 hours. Then, Compound B dissolved in 20 mL of dehydrated diethyl ether was added dropwise thereto, followed by stirring overnight. After the reaction was complete, water was added, an organic layer was isolated, and the solvents were removed via distillation. The crude product thus obtained was separated by silica gel chromatography (using toluene) to obtain 2.20 g (yield 62%) of Intermediate 1A as a white solid.

The molecular weight of Intermediate 1A, as measured by FAB-MS, was 440.

### Synthesis of Intermediate 2A

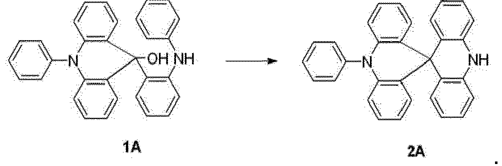

2.0 g of Compound 1A and 20 mL of toluene were added to a 100 mL, three-neck flask under an argon (Ar) atmosphere, and 0.73 g of methylsulfonic acid and 1.0 g of polyphosphoric acid were added thereto, followed by heating, refluxing, and stirring for about 4 hours. After the reaction was complete, water was added, an organic layer was isolated, and the solvents were removed via distillation. The crude product thus obtained was separated by silica gel chromatography (using toluene) to obtain 1.9 g (yield 95%) of Intermediate 2A as a white solid.

The molecular weight of Intermediate 2A, as measured by FAB-MS, was 422.

### Synthesis of Final Compound 8

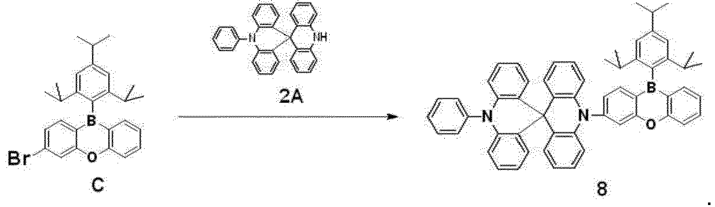

1.0 g of Compound C, 0.95 g of Intermediate 2A, 0.43 g of sodium tert-butoxide, 0.04 g of tris(dibenzylideneacetone)dipalladium(0), 0.05 g of tri-tert-butylphosphonium tetrafluoroborate, and 20 mL of toluene were added to a 100 mL, three-neck flask under an argon (Ar) atmosphere, followed by heating, refluxing, and stirring for about 8 hours. After the reaction was complete, water was added, an organic layer was isolated, and the solvents were removed via distillation. The crude product thus obtained was separated by silica gel chromatography (using a mixture of toluene and hexane) to obtain 1.7 g (yield 94%) of Compound 8 as a white solid.

The molecular weight of Compound 8, as measured by FAB-MS, was 802.

### 2. Synthesis of Compound 69

Compound 69 according to one or more embodiments of the present disclosure may be synthesized, for example, as follows.

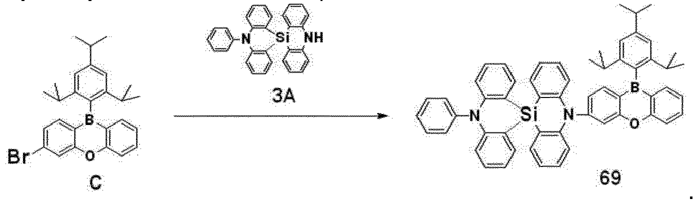

Compound 69 was obtained in 89% yield by conducting substantially the same procedure as described in the synthetic method of Compound 8, except for using Intermediate 3A instead of Intermediate 2A. The molecular weight of Compound 69, as measured by FAB-MS, was 818.

The above-described synthetic examples are illustrations, and reaction conditions may be changed as necessary. The compounds according to one or more embodiments of the present disclosure may be synthesized so as to include various suitable substituents using methods and materials available in the art. Such substituents may be introduced into a core structure represented by Formula 1 to yield properties suitable for application to an organic electroluminescence device.

### Device manufacturing examples

Organic electroluminescence devices of Examples 1 and 2 were manufactured using Compounds 8 and 69, respectively, as dopant materials in the emission layer of each device.

### Example Compounds

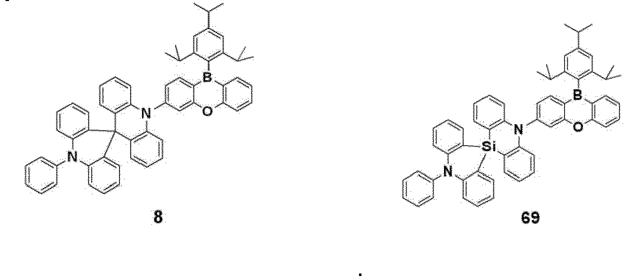

Organic electroluminescence devices of Comparative Examples 1 to 10 were manufactured using Compounds C-1 to C-10, respectively, as dopant materials in the emission layer of each device.

### Comparative Compounds

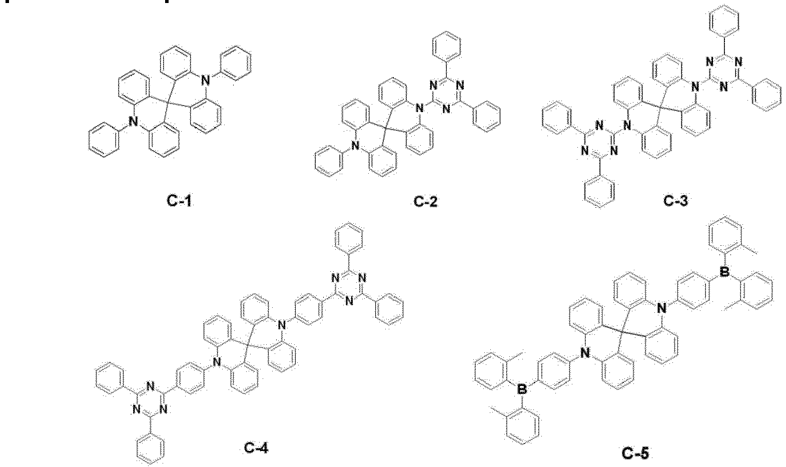

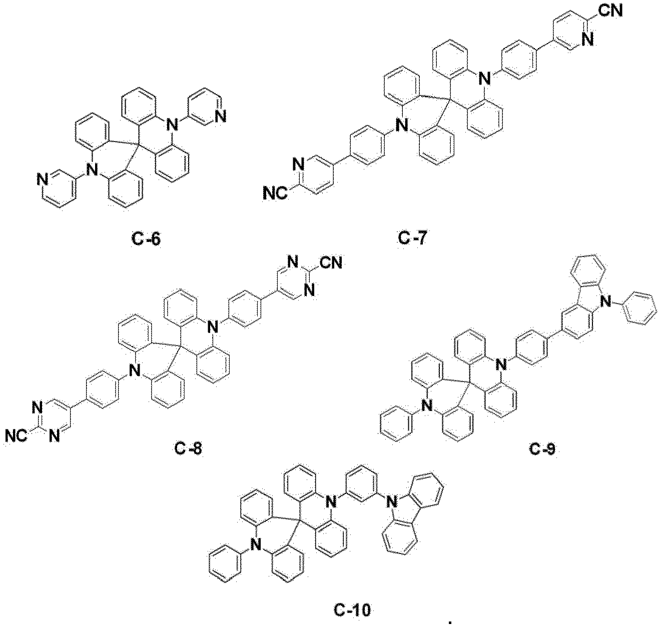

The S₁ (singlet) energy levels and T₁ (triplet) energy levels of the example compounds and the comparative compounds were calculated using a non-empirical molecular orbital method. For example, the calculations were conducted using B3LYP as a functional, and 6-31 G(d) as a basis function in Gaussian 09 (Gaussian Co., Wallingford, CT). The results are shown in Table 1. ΔE_{ST} represents the difference between the singlet energy level and the triplet energy level.

**Table 1**

| | S₁ energy level (eV) | T₁ energy level (eV) | ΔE_{ST} |
|---|---|---|---|
| Example Compound 8 | 2.56 | 2.55 | 0.01 |
| Example Compound 69 | 2.72 | 2.71 | 0.01 |
| Comparative Compound C-1 | 3.67 | 3.15 | 0.52 |
| Comparative Compound C-2 | 2.96 | 2.96 | 0.00 |
| Comparative Compound C-3 | 3.48 | 3.15 | 0.33 |
| Comparative Compound C-4 | 2.41 | 2.41 | 0.00 |
| Comparative Compound C-5 | 2.46 | 2.46 | 0.00 |
| Comparative Compound C-6 | 3.30 | 3.13 | 0.17 |
| Comparative Compound C-7 | 2.54 | 2.53 | 0.01 |
| Comparative Compound C-8 | 2.36 | 2.35 | 0.01 |
| Comparative Compound C-9 | 3.38 | 3.06 | 0.32 |
| Comparative Compound C-10 | 3.49 | 3.14 | 0.35 |

Referring to Table 1, Example Compounds 8 and 69, and Comparative Compounds C-2 and C-4 to C-8 each showed low ΔE_{ST} values, and are thought to exhibit thermally activated delayed fluorescence. Comparative Compounds C-1, C-3, C-9 and C-10 showed high ΔE_{ST} values, and are not thought to exhibit thermally activated delayed fluorescence. Each organic electroluminescence device of Examples 1 and 2 and Comparative Examples 1 to 10 was manufactured by forming a first electrode using ITO to a thickness of about 150 nm, a hole injection layer using HAT-CN to a thickness of about 10 nm, a hole transport layer using NPB to a thickness of about 80 nm, an electron blocking layer using mCP to a thickness of about 5 nm, an emission layer using DPEPO doped with 20% of the Example Compound or the Comparative Compound to a thickness of about 20 nm, a hole blocking layer using DPEPO to a thickness of about 10 nm, an electron transport layer using TPBi to a thickness of about 30 nm, an electron injection layer using LiF to a thickness of about 0.5 nm, and a second electrode using Al to a thickness of about 100 nm. Each layer was formed using a vacuum deposition method.

The maximum emission wavelength and external quantum efficiency of each of the organic electroluminescence devices according to Examples 1 and 2 and Comparative Examples 1 to 10 were measured and are shown in Table 2. The EQE values in Table 2 were measured at about 10 mA/cm².

**Table 2**

| | Emission layer dopant material | λₘₐₓ (nm) | EQE (%) |
|---|---|---|---|
| Example 1 | Example Compound 8 | 468 | 23.8 |
| Example 2 | Example Compound 69 | 459 | 22.5 |
| Comparative Example 1 | Comparative Compound C-1 | 413 | 1.2 |
| Comparative Example 2 | Comparative Compound C-2 | 489 | 3.2 |
| Comparative Example 3 | Comparative Compound C-3 | 496 | 2.8 |
| Comparative Example 4 | Comparative Compound C-4 | 501 | 17.5 |
| Comparative Example 5 | Comparative Compound C-5 | 498 | 15.1 |
| Comparative Example 6 | Comparative Compound C-6 | 420 | 2.1 |
| Comparative Example 7 | Comparative Compound C-7 | 470 | 5.5 |
| Comparative Example 8 | Comparative Compound C-8 | 512 | 8.2 |
| Comparative Example 9 | Comparative Compound C-9 | 415 | 1.1 |
| Comparative Example 10 | Comparative Compound C-10 | 412 | 1.3 |

Referring to the results of Table 2, Example Compounds 8 and 69 all had light emission wavelengths of less than about 470 nm, and are suitable as deep blue dopants, thereby accomplishing high efficiency due to thermally activated delayed fluorescence. Comparative Examples 1, 6, 9 and 10 showed relatively low emission efficiencies and short emission wavelengths. Comparative Examples 4, 5 and 8 showed relatively high efficiencies but longer wavelengths, and deep blue light emission was not accomplished. Comparative Example 7 showed a wavelength near a deep blue color but with lower efficiency than the examples. Comparative Example 2 showed neither a deep blue color nor high efficiency. Though ΔE_{ST} of the calculated value of Comparative Example 2 was small, there was a chance that thermally activated delayed fluorescence was not shown. Comparative Example 3 showed a low efficiency and longer wavelength, and did not show a deep blue color.

The polycyclic compound according to one or more embodiments of the present disclosure may be utilized as a dopant of thermally activated delayed fluorescence for emitting deep blue color.

The organic electroluminescence device including the polycyclic compound according to one or more embodiments of the present disclosure accomplishes deep blue color and has excellent efficiency at the same time.

The organic electroluminescence device according to one or more embodiments of the present disclosure has excellent efficiency.

The polycyclic compound according to one or more embodiments of the present disclosure may be applied to an organic electroluminescence device and may contribute to high efficiency.

As used herein, expressions such as "at least one of", "one of", and "selected from", when preceding a list of elements, modify the entire list of elements and do not modify the individual elements of the list. Further, the use of "may" when describing embodiments of the present disclosure refers to "one or more embodiments of the present disclosure".

In addition, as used herein, the terms "use", "using", and "used" may be considered synonymous with the terms "utilize", "utilizing", and "utilized", respectively.

As used herein, the terms "substantially", "about", and similar terms are used as terms of approximation and not as terms of degree, and are intended to account for the inherent deviations in measured or calculated values that would be recognized by those of ordinary skill in the art.

Also, any numerical range recited herein is intended to include all subranges of the same numerical precision subsumed within the recited range. For example, a range of "1.0 to 10.0" is intended to include all subranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited herein is intended to include all lower numerical limitations subsumed therein and any minimum numerical limitation recited in this specification is intended to include all higher numerical limitations subsumed therein.

Although example embodiments of the present disclosure have been described, it is understood that the present disclosure should not be limited to these example embodiments, but that various changes and modifications can be made by one ordinary skilled in the art within the scope of the present disclosure, as described in the following claims and equivalents thereof.

## Claims

1. A polycyclic compound represented by Formula 1: 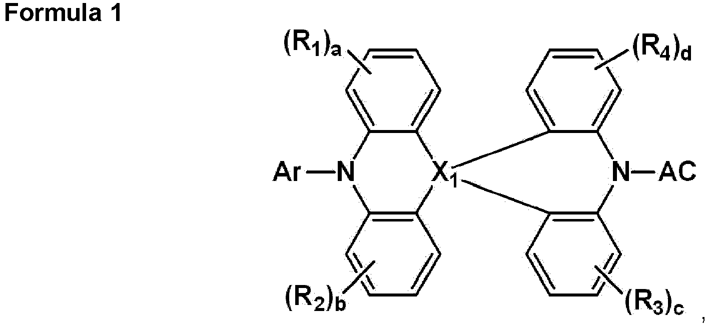 wherein, in Formula 1,
X₁ is C, Si, or Ge,
Ar is a substituted or unsubstituted aryl group having 6 to 30 carbon atoms for forming a ring, or a substituted or unsubstituted heteroaryl group having 2 to 30 carbon atoms for forming a ring,
R₁ to R₄ are each independently a hydrogen atom, a deuterium atom, a substituted or unsubstituted alkyl group having 1 to 10 carbon atoms, a substituted or unsubstituted aryl group having 6 to 30 carbon atoms for forming a ring, or a substituted or unsubstituted heteroaryl group having 2 to 30 carbon atoms for forming a ring,
"a" to "d" are each independently an integer of 0 to 4, and
AC is represented by Formula 3: 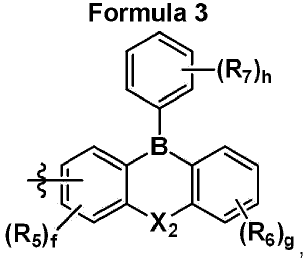 wherein, in Formula 3,
X₂ is O, S or CR₈R₉,
R₅ to R₉ are each independently a hydrogen atom, a deuterium atom, or a substituted or unsubstituted alkyl group having 1 to 5 carbon atoms, or are combined with an adjacent group to form a hydrocarbon ring or a heterocycle,
"f" is an integer of 0 to 3,
"g" is an integer of 0 to 4, and
"h" is an integer of 0 to 5.

2. A polycyclic compound according to claim 1, wherein Formula 3 is further represented by Formula 3-1 or 3-2: 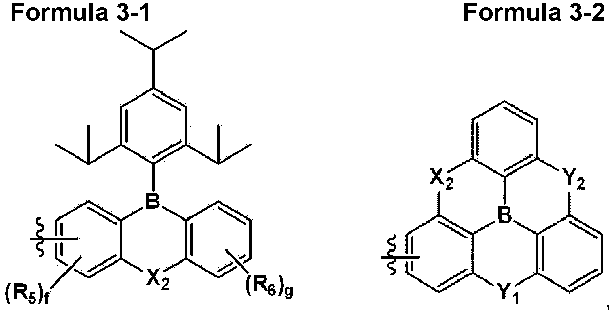 wherein, in Formulae 3-1 and 3-2,
X₂, R₅, R₆, "f" and "g" are each independently the same as defined in Formula 3,
Y₁ and Y₂ are each independently O, S or CR₂₆R₂₇, and
R₂₆ and R₂₇ are each independently a hydrogen atom, a deuterium atom, or a substituted or unsubstituted alkyl group having 1 to 5 carbon atoms.

3. A polycyclic compound according to claim 1, wherein the polycyclic compound represented by Formula 1 is at least one selected from compounds represented in Compound Group 1:

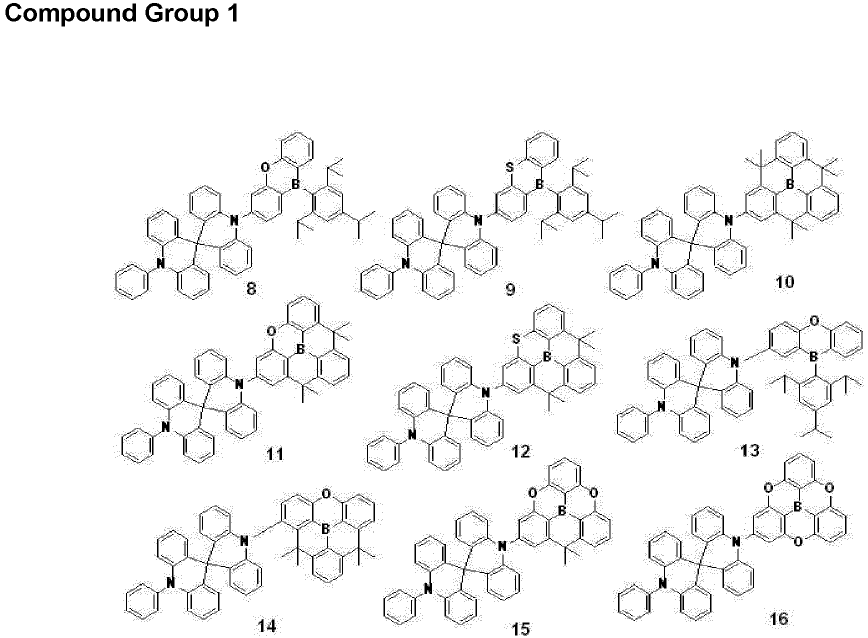

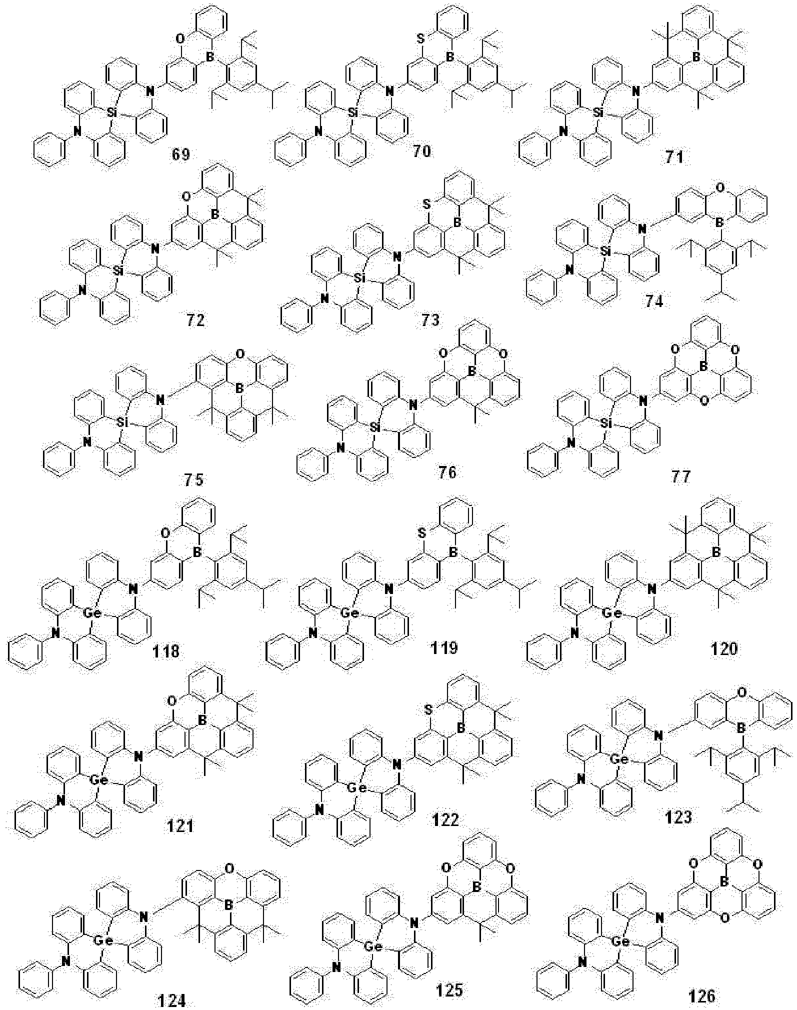

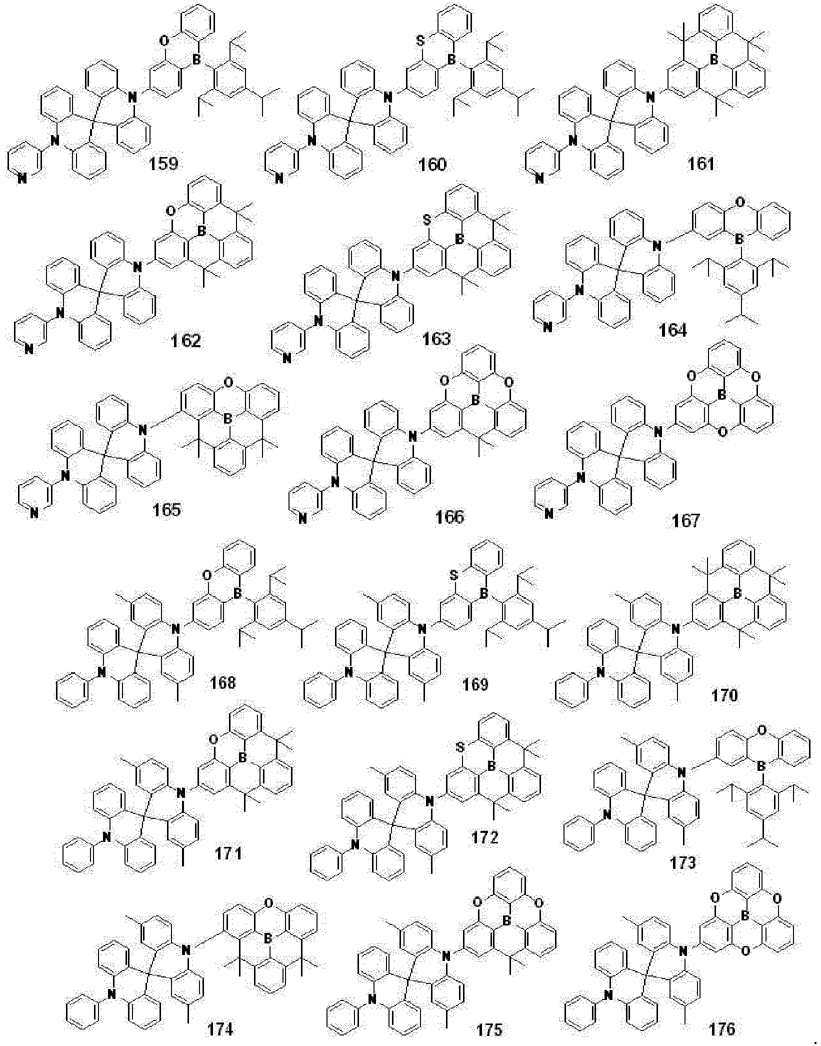

4. An organic electroluminescence device, comprising:
a first electrode;
a hole transport region on the first electrode;
an emission layer on the hole transport region and comprising a polycyclic compound according to any one of claims 1 to 3;
an electron transport region on the emission layer; and
a second electrode on the electron transport region, wherein the first electrode and the second electrode each independently comprise at least one selected from Ag, Mg, Cu, Al, Pt, Pd, Au, Ni, Nd, Ir, Cr, Li, Ca, LiF/Ca, LiF/AI, Mo and Ti, a compound thereof, a mixture thereof, indium tin oxide, indium zinc oxide, zinc oxide and indium tin zinc oxide.

5. An organic electroluminescence device according to claim 4, wherein Ar is a substituted or unsubstituted phenyl group.

6. An organic electroluminescence device according to claim 4 or claim 5, wherein the emission layer comprises a host and a dopant, and
the dopant comprises the polycyclic compound.

7. An organic electroluminescence device according to claim 6, wherein the polycyclic compound is a dopant for thermally activated delayed fluorescence.

8. An organic electroluminescence device according to claim 6 or claim 7, wherein the polycyclic compound is a blue dopant having a wavelength of less than about 470 nm.

9. An organic electroluminescence device according to any one of claims 4 to 8, wherein the hole transport region comprises:
a hole injection layer on the first electrode;
a hole transport layer on the hole injection layer; and
an electron blocking layer on the hole transport layer.

10. An organic electroluminescence device according to any one of claims 4 to 9, wherein the electron transport region comprises:
a hole blocking layer on the emission layer;
an electron transport layer on the hole blocking layer; and
an electron injection layer on the electron transport layer.

## Patentansprüche

1. Polycyclische Verbindung, dargestellt durch Formel 1: 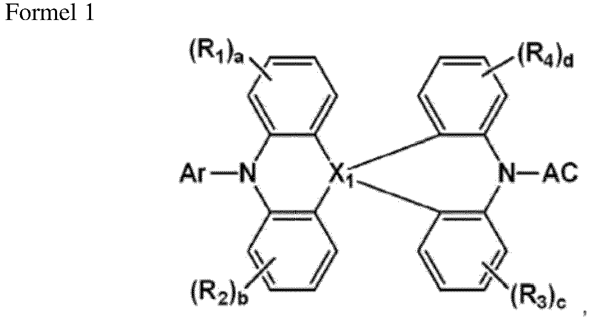 wobei, in Formel 1,
X₁ C, Si oder Ge ist,
Ar eine substituierte oder unsubstituierte Arylgruppe mit 6 bis 30 Kohlenstoffatomen zur Bildung eines Rings ist, oder eine substituierte oder unsubstituierte Heteroarylgruppe mit 2 bis 30 Kohlenstoffatomen zur Bildung eines Rings,
R₁ bis R₄ jeweils unabhängig ein Wasserstoffatom, ein Deuteriumatom, eine substituierte oder unsubstituierte Alkylgruppe mit 1 bis 10 Kohlenstoffatomen, eine substituierte oder unsubstituierte Arylgruppe mit 6 bis 30 Kohlenstoffatomen zur Bildung eines Rings, oder eine substituierte oder unsubstituierte Heteroarylgruppe mit 2 bis 30 Kohlenstoffatomen zur Bildung eines Rings sind,
"a" bis "d" jeweils unabhängig eine ganze Zahl von 0 bis 4 sind, und
AC durch Formel 3 dargestellt ist: 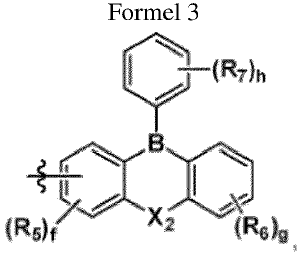 wobei, in Formel 3,
X₂ O, S oder CR₈R₉ ist,
R₅ bis R₉ jeweils unabhängig ein Wasserstoffatom, ein Deuteriumatom, oder eine substituierte oder unsubstituierte Alkylgruppe mit 1 bis 5 Kohlenstoffatomen sind, oder mit einer benachbarten Gruppe zwecks Bildung eines Kohlenwasserstoffrings oder eines Heterocyclus kombiniert sind,
"f" eine ganze Zahl von 0 bis 3 ist,
"g" eine ganze Zahl von 0 bis 4 ist, und
"h" eine ganze Zahl von 0 bis 5 ist.

2. Polycyclische Verbindung nach Anspruch 1, wobei Formel 3 weiterhin durch Formel 3-1 oder 3-2 dargestellt ist: 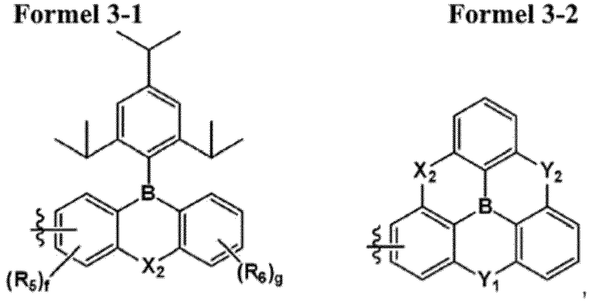 wobei, in Formeln 3-1 und 3-2,
X₂, R₅, R₆, "f" und "g" jeweils unabhängig so sind wie in Formel 3 definiert,
Y₁ und Y₂ jeweils unabhängig O, S oder CR₂₆R₂₇ sind, und
R₂₆ und R₂₇ jeweils unabhängig ein Wasserstoffatom, ein Deuteriumatom, oder eine substituierte oder unsubstituierte Alkylgruppe mit 1 bis 5 Kohlenstoffatomen sind.

3. Polycyclische Verbindung nach Anspruch 1, wobei die durch Formel 1 dargestellte polycyclische Verbindung zumindest eine ist, die aus Verbindungen ausgewählt ist, die in Verbindungsgruppe 1 dargestellt sind:

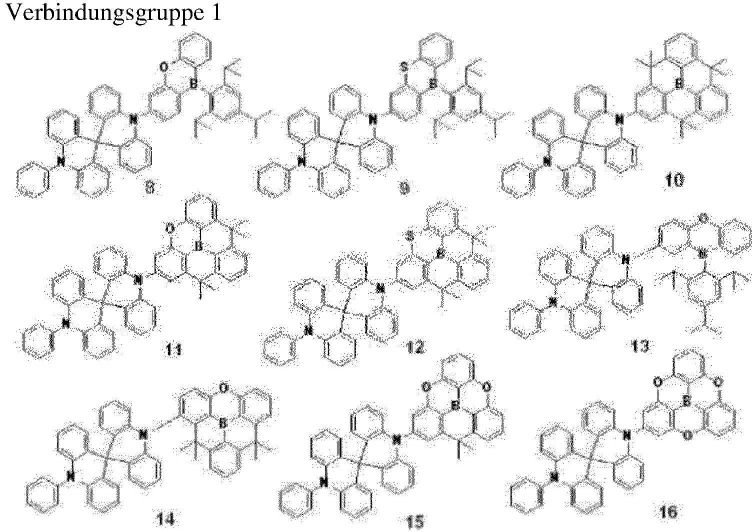

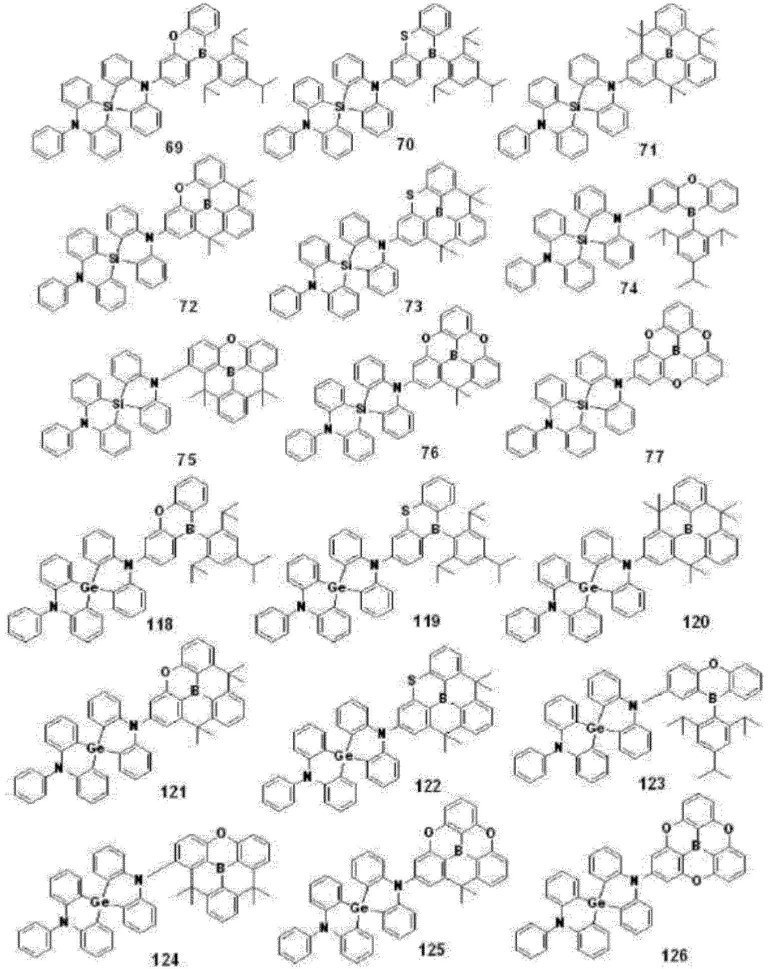

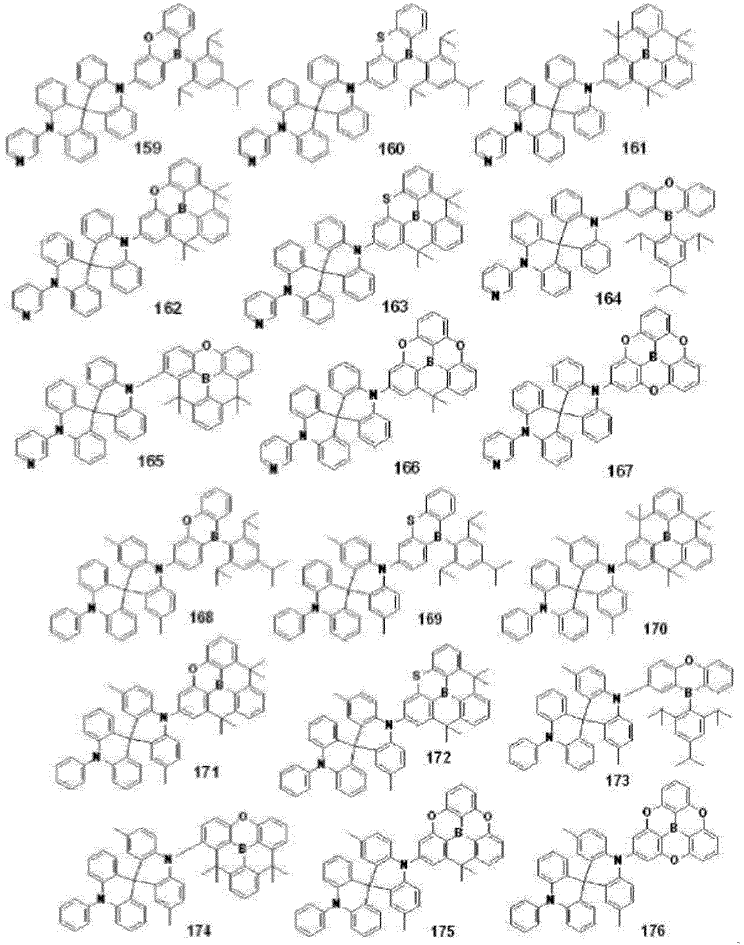

4. Organische Elektrolumineszenzvorrichtung, umfassend:
eine erste Elektrode;
eine Löchertransportregion auf der ersten Elektrode;
eine Emissionsschicht auf der Löchertransportregion und umfassend eine polycyclische Verbindung nach einem der Ansprüche 1 bis 3;
eine Elektronentransportregion auf der Emissionsschicht; und
eine zweite Elektrode auf der Elektronentransportregion, wobei die erste Elektrode und die zweite Elektrode jeweils unabhängig zumindest eines umfassen, das ausgewählt ist aus Ag, Mg, Cu, Al, Pt, Pd, Au, Ni, Nd, Ir, Cr, Li, Ca, LiF/Ca, LiF/Al, Mo und Ti, einer Verbindung davon, einem Gemisch davon, Indiumzinnoxid, Indiumzinkoxid, Zinkoxid und Indiumzinnzinkoxid.

5. Organische Elektrolumineszenzvorrichtung nach Anspruch 4, wobei Ar eine substituierte oder unsubstituierte Phenylgruppe ist.

6. Organische Elektrolumineszenzvorrichtung nach Anspruch 4 oder Anspruch 5, wobei die Emissionsschicht einen Wirt und ein Dotierungsmittel umfasst, und
das Dotierungsmittel die polycyclische Verbindung umfasst.

7. Organische Elektrolumineszenzvorrichtung nach Anspruch 6, wobei die polycyclische Verbindung ein Dotierungsmittel für thermisch aktivierte verzögerte Fluoreszenz ist.

8. Organische Elektrolumineszenzvorrichtung nach Anspruch 6 oder Anspruch 7, wobei die polycyclische Verbindung ein Blaudotierungsmittel mit einer Wellenlänge unter ungefähr 470 nm ist.

9. Organische Elektrolumineszenzvorrichtung nach einem der Ansprüche 4 bis 8, wobei die Löchertransportregion umfasst:
eine Löcherinjektionsschicht auf der ersten Elektrode;
eine Löchertransportschicht auf der Löcherinjektionsschicht; und
eine Elektronenblockierschicht auf der Löchertransportschicht.

10. Organische Elektrolumineszenzvorrichtung nach einem der Ansprüche 4 bis 9, wobei die Elektronentransportregion umfasst:
eine Löcherblockierschicht auf der Emissionsschicht;
eine Elektronentransportschicht auf der Löcherblockierschicht; und
eine Elektroneninjektionsschicht auf der Elektronentransportschicht.

## Revendications

1. Composé polycyclique représenté par la Formule 1 : 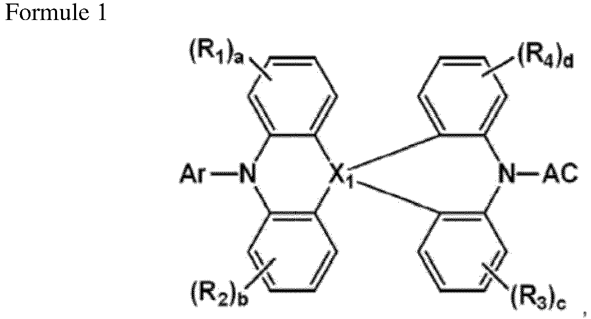 dans lequel, dans la Formule 1,
X₁ est C, Si ou Ge,
Ar est un groupe aryle substitué ou non substitué possédant de 6 à 30 atomes de carbone pour la formation d'un cycle ou un groupe hétéroaryle substitué ou non substitué possédant de 2 à 30 atomes de carbone pour la formation d'un cycle,
R₁ à R₄ sont chacun indépendamment un atome d'hydrogène, un atome de deutérium, un groupe alkyle substitué ou non substitué possédant de 1 à 10 atomes de carbone, un groupe aryle substitué ou non substitué possédant de 6 à 30 atomes de carbone pour la formation d'un cycle ou un groupe hétéroaryle substitué ou non substitué possédant de 2 à 30 atomes de carbone pour la formation d'un cycle,
« a » à « d » sont chacun indépendamment un nombre entier de 0 à 4, et
AC est représenté par la Formule 3 : 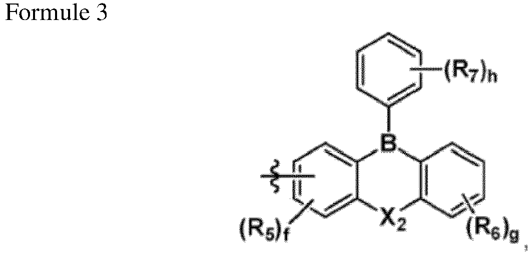 dans lequel, dans la Formule 3,
X₂ est O, S ou CR₈R₉,
R₅ à R₉ sont chacun indépendamment un atome d'hydrogène, un atome de deutérium ou un groupe alkyle substitué ou non substitué possédant de 1 à 5 atomes de carbone, ou sont combinés avec un groupe adjacent pour former un cycle hydrocarbure ou un hétérocycle,
« f » est un nombre entier de 0 à 3,
« g » est un nombre entier de 0 à 4, et
« h » est un nombre entier de 0 à 5.

2. Composé polycyclique selon la revendication 1, dans lequel la Formule 3 est en outre représentée par la Formule 3-1 ou 3-2 : 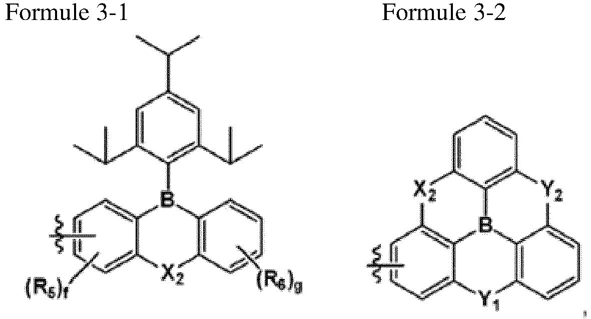 dans lequel, dans les Formules 3-1 et 3-2,
X₂, R₅, R₆, « f » et « g » sont chacun indépendamment identiques à ce qui est défini dans la Formule 3,
Y₁ et Y₂ sont chacun indépendamment O, S ou CR₂₆R₂₇, et
R₂₆ et R₂₇ sont chacun indépendamment un atome d'hydrogène, un atome de deutérium ou un groupe alkyle substitué ou non substitué possédant de 1 à 5 atomes de carbone.

3. Composé polycyclique selon la revendication 1, où le composé polycyclique représenté par la Formule 1 est au moins un choisi parmi les composés représentés dans le Groupe de composés 1 :

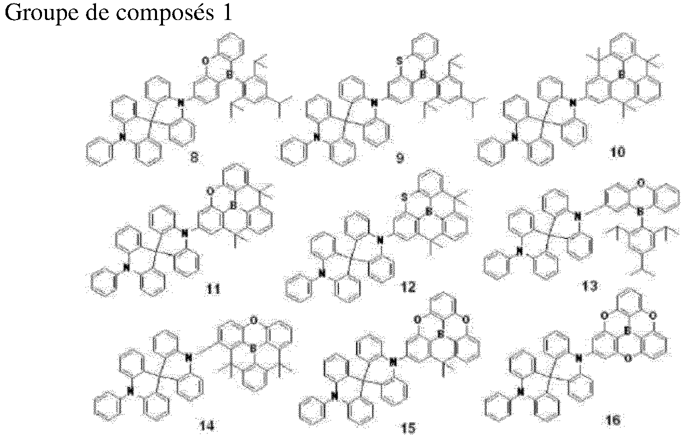

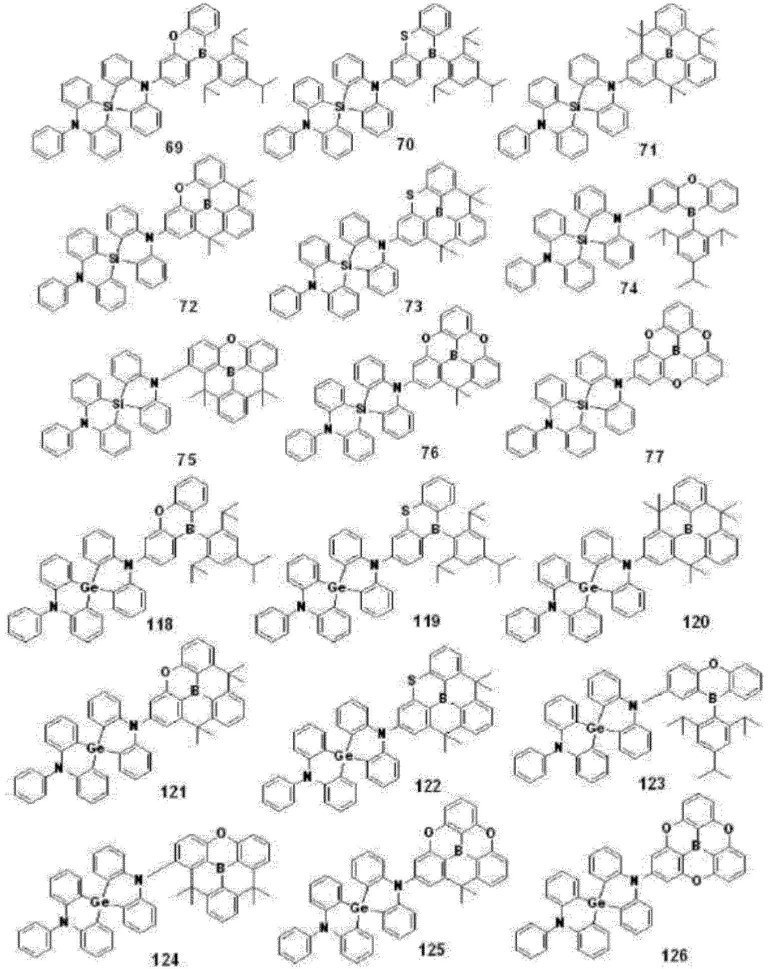

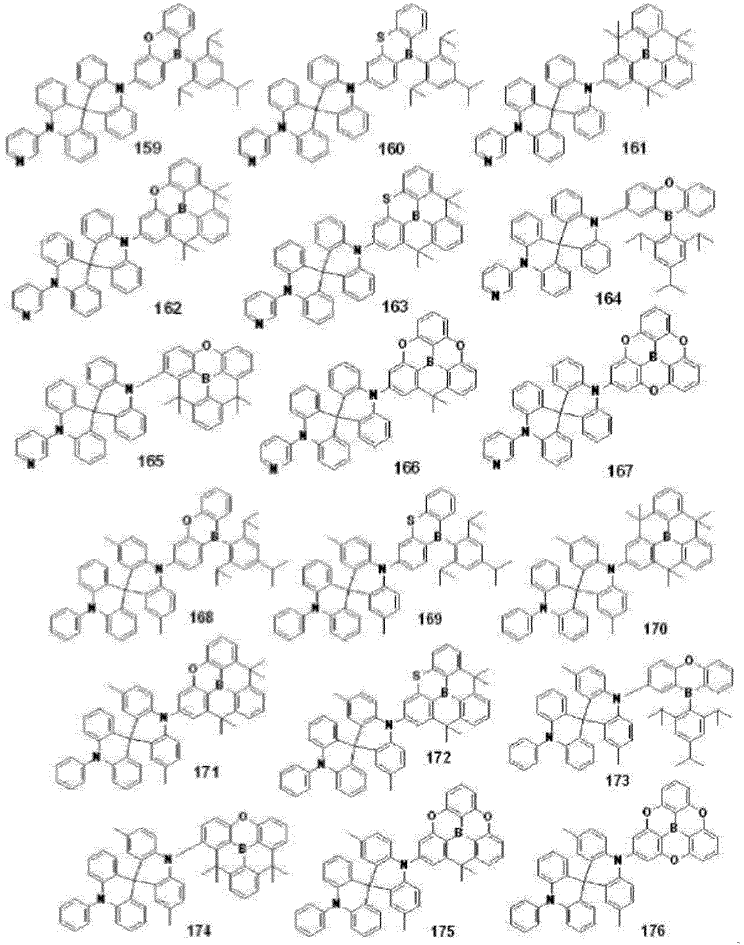

4. Dispositif électroluminescent organique, comprenant :
une première électrode ;
une région de transport de trous sur la première électrode ;
une couche d'émission sur la région de transport de trous et comprenant un composé polycyclique selon l'une quelconque des revendications 1 à 3 ;
une région de transport d'électrons sur la couche d'émission ; et
une deuxième électrode sur la région de transport d'électrons, dans lequel la première électrode et la deuxième électrode comprennent chacune indépendamment au moins un choisi parmi Ag, Mg, Cu, Al, Pt, Pd, Au, Ni, Nd, Ir, Cr, Li, Ca, LiF/Ca, LiF/Al, Mo et Ti, un composé de ceux-ci, un mélange de ceux-ci, un oxyde d'indium et d'étain, un oxyde d'indium et de zinc, un oxyde de zinc et un oxyde d'indium, d'étain et de zinc.

5. Dispositif électroluminescent organique selon la revendication 4, dans lequel Ar est un groupe phényle substitué ou non substitué.

6. Dispositif électroluminescent organique selon la revendication 4 ou la revendication 5, dans lequel la couche d'émission comprend un hôte et un dopant, et
le dopant comprend le composé polycyclique.

7. Dispositif électroluminescent organique selon la revendication 6, dans lequel le composé polycyclique est un dopant pour une fluorescence retardée activée thermiquement.

8. Dispositif électroluminescent organique selon la revendication 6 ou la revendication 7, dans lequel le composé polycyclique est un dopant bleu ayant une longueur d'onde de moins d'environ 470 nm.

9. Dispositif électroluminescent organique selon l'une quelconque des revendications 4 à 8, dans lequel la région de transport de trous comprend :
une couche d'injection de trous sur la première électrode ;
une couche de transport de trous sur la couche d'injection de trous ; et
une couche de blocage d'électrons sur la couche de transport de trous.

10. Dispositif électroluminescent organique selon l'une quelconque des revendications 4 à 9, dans lequel la région de transport d'électrons comprend :
une couche de blocage de trous sur la couche d'émission ;
une couche de transport d'électrons sur la couche de blocage de trous ; et
une couche d'injection d'électrons sur la couche de transport d'électrons.
